# EUROPÄISCHE PATENTSCHRIFT

(11) **EP 4 021 346 B1**
(45) Veröffentlichungstag und Bekanntmachung des Hinweises auf die Patenterteilung: **04.10.2023**
(21) Anmeldenummer: 20753740.8
(22) Anmeldetag: 07.08.2020
(51) Int. Cl.: A61F 2/16

(54) **INTRAOKULARLINSE MIT SPEZIFISCHER, DREIDIMENSIONAL GEKRÜMMTER HAPTIK**
INTRAOCULAR LENS HAVING A SPECIFIC, THREE-DIMENSIONALLY CURVED HAPTIC ELEMENT
LENTILLE INTRAOCCULAIRE ÉQUIPÉE D'UNE STRUCTURE HAPTIQUE SPÉCIFIQUE À COURBURE TRIDIMENSIONNELLE

(30) Priorität: 30.08.2019 DE 102019123295
(43) Veröffentlichungstag der Anmeldung: 06.07.2022
(73) Patentinhaber: Carl Zeiss Meditec AG, 07745 Jena (DE)
(72) Erfinder: MÜLLER, Stephanie, 16816 Neuruppin (DE)
(74) Vertreter: Hofstetter, Schurack & Partner
(86) Internationale Anmeldenummer: PCT/EP2020/072215
(87) Internationale Veröffentlichungsnummer: WO 2021/037523

(56) Entgegenhaltungen:
- DE-A1- 10 016 929
- DE-A1-102007 057 122
- US-A- 4 804 361
- US-A1- 2005 113 914
- US-A1- 2007 100 444
- US-A1- 2013 053 955
- US-A1- 2015 142 108

## Beschreibung

### Technisches Gebiet

Ein Aspekt der Erfindung betrifft eine Intraokularlinse mit einem einzigen optischen Teil und mit einer Haptik, die mit dem optischen Teil gekoppelt ist, und mit einer optischen Hauptachse, die eine Vorderseite und eine Rückseite des optischen Teils durchdringt, wobei die Haptik ein erstes Haptikteil aufweist, welches als erster Ring und umlaufend um den optischen Teil ausgebildet ist, und zumindest ein zweites Haptikteil aufweist, welches als zweiter Ring und umlaufend um den optischen Teil ausgebildet ist und welches relativ zum ersten Haptikteil elastisch bewegbar ist, wobei zumindest einer der beiden Ringe in Umlaufrichtung um die optische Hauptachse uneben ausgebildet ist,

### Stand der Technik

Intraokularlinsen sind in vielfältigen Ausgestaltungen bekannt. Üblicherweise weisen Intraokularlinsen zumindest zwei separate Haptiken auf, die in Umlaufrichtung um die optische Hauptachse gegenüberliegend ausgebildet sind und radial anschließend an den optischen Teil ausgebildet sind. Es können auch mehr als zwei derartige separate Haptiken ausgebildet sein, beispielsweise drei Haptiken.

Intraokularlinsen können an unterschiedlichen definierten Positionen im Auge anstelle einer natürlichen Linse des Auges implantiert werden. So ist es in dem Zusammenhang vorgesehen, dass spezifische Intraokularlinsen in einer Vorderkammer des Auges implantiert werden. Beispielsweise können derartige Vorderkammerlinsen im vorderen Kammerwinkel fixiert werden.

Darüber hinaus sind Intraokularlinsen bekannt, die als Iris-Clip-Linsen bezeichnet werden. Derartige Intraokularlinsen werden an der Pupille befestigt. Insbesondere werden sie dabei an die Pupillenöffnung geklemmt. Eine derartige Intraokularlinse ist beispielsweise aus der DE 10 2007 057 122 A1 bekannt. Die dortige Intraokularlinse mit diesem spezifischen Implantationsort im Auge weist zwei gegenüberliegende Haptiken auf. Jede dieser Haptiken weist zwei L-förmig geformte Haptikarme auf. Einander zugewandte Enden dieser Haptikarme sind, in einer Ebene senkrecht zur optischen Hauptachse dieser Intraokularlinse betrachtet, einander zugewandt angeordnet, jedoch berührungslos und überlappungsfrei angeordnet. Mittels derartig gebildeter Haptikarme kann durch den gebildeten Spalt, der in Umlaufrichtung um die optische Hauptachse zwischen den Enden der Haptikarme gebildet ist, die Iris eingeklemmt werden. Derartige Linsen sind jedoch nicht dazu vorgesehen und tauglich, in einem Kapselsack eines Auges implantiert werden zu können.

Diesbezüglich sind weitere spezifische Intraokularlinsen bekannt, die als Hinterkammerlinsen bezeichnet werden können und in einen Kapselsack des Auges implantiert werden.

Aus der DE 103 10 961 B4 ist eine Intraokularlinse bekannt, die eine Hinterkammerlinse ist. Bei dieser Hinterkammerlinse ist vorgesehen, dass zwei separate Haptiken an gegenüberliegenden Bereichen des optischen Teils radial anschließend an den optischen Teil ausgebildet sind. Beide jeweiligen Haptiken sind mit zwei haptischen Teilen ausgebildet. Die beiden haptischen Teile einer Haptik sind relativ zueinander bewegbar. Dazu ist an einer definierten Verbindungsstelle zwischen den einstückig miteinander verbundenen Haptikteilen eine definierte Knickstelle, beispielsweise als Filmscharnier, ausgebildet. Dadurch kann das radial äußere haptische Teil dieser Haptik gegenüber dem ersten haptischen Teil, welches direkt an dem optischen Teil anschließt, geknickt beziehungsweise verklappt werden. Diese Klappbewegung ist nur in der Ebene senkrecht zur optischen Achse möglich. Dadurch soll dort die radiale Weite der gesamten Intraokularlinse reduziert werden, um Reizungen im Inneren des Kapselsacks durch diese Haptiken vermeiden zu können.

Derartige Hinterkammerlinsen haben eine mehr oder weniger planare Struktur. Dies hat zur Folge, dass der nach der Implantation schrumpfende Kapselsack mit der hinteren Seite des optischen Teils in Berührung kommt. An der Berührungsfläche kann es zur Migration von Zellen kommen, sodass sich der hintere Kapselsack eintrüben kann. Bekannt ist es, dass sich eine derartige Eintrübung des hinteren Kapselsacks dadurch vermeiden lässt oder zumindest reduzieren lässt, wenn die Rückseite des optischen Teils der Intraokularlinse vom Kammerwasser umspült wird. Dies erfordert, dass ein Abstand zwischen dieser Rückseite des optischen Teils die Intraokularlinse und dem hinteren Kapselsack vorhanden ist.

Eine Beabstandung einer Rückseite des optischen Teils der Intraokularlinse zu dem Kapselsack im implantierten Zustand der Intraokularlinse im Kapselsack ist bei der DE 103 10 961 B4 nicht möglich. Daher wird auch durch diese Hinterkammerlinse die oben genannte Problemstellung nicht gelöst.

Aus der US 2007/0100444 A1 ist eine Intraokularlinse bekannt, die eine dreidimensional geformte Haptik aufweist, die als Geflecht ausgebildet ist. Die Haptik weist mehrere Bögen auf, die sich radial zur optischen Hauptachse ausbauchen. Diese Bögen sind mit ihren beiden Enden an unterschiedlichen Positionen in Richtung der optischen Hauptachse der Intraokularlinse angeordnet. Ein Ende der Bögen ist an einer Umfangsseite des optischen Teils der Intraokularlinse betrachtet angeordnet. Das zweite Ende der Bögen ist axial nach vorne über den optischen Teil überstehend und an einem Kreisring der Haptik befestigt. Der Kreisring ist vollständig in einer Flächenebene angeordnet, die entfernt zum optischen Teil angeordnet ist und senkrecht zur optischen Achse der Linse orientiert ist.

Aus der US 8 043 372 B2 ist eine Intraokularlinse bekannt die ebenfalls eine dreidimensional geformte Haptik aufweist, die sich mit ihrer Form nicht in einer Ebene erstreckt. Diese Haptik weist in einem Ausführungsbeispiel zwei um die optische Hauptachse umlaufende Ringe auf. Diese Ringe sind jeweils mäanderförmig gewellt. Die Mäanderform mit ihren vielen Schlingen ist um die optische Hauptachse umlaufend ausgebildet. Die beiden Ringe sind an Wellenumkehrpunkten miteinander verbunden, wobei die radial weiter innen liegenden Ringberge frei auskragend sind und einander zugewandt sind beziehungsweise der optischen Hauptachse zugewandt sind.

Durch derartige dreidimensionale Haptiken soll das sichere Halten der Intraokularlinse im Kapselsack verbessert werden. Diese Haptiken sind jedoch sehr formkomplex und daher schwierig herzustellen. Durch die Formkomplexität ist auch das Implantieren der Intraokularlinse in den Kapselsack erschwert. Des Weiteren besteht bei diesen Haptiken auch das Problem, das sie aufgrund ihrer Symmetrie um die optische Hauptachse in

einem Kapselsack bereichsweise radial stark gestaucht werden.

Eine Intraokularlinse nach dem Oberbegriff von Anspruch 1 ist bekannt aus US 4 804 361 A.

### Darstellung der Erfindung

Es ist Aufgabe der vorliegenden Erfindung, eine Intraokularlinse zu schaffen, die eine verbesserte Positionierung in einem Kapselsack eines Auges ermöglicht.

Diese Aufgabe wird durch eine künstliche Intraokularlinse gemäß den Merkmalen des unabhängigen Anspruchs gelöst.

Ein Aspekt der Erfindung betrifft eine künstliche Intraokularlinse mit einem einzigen optischen Teil. Der optische Teil ist eine Linse. Der optische Teil weist spezifische optische Abbildungseigenschaften auf, sodass damit spezifische Korrekturen von Sehfehlern durchgeführt werden können.

Die Intraokularlinse weist darüber hinaus eine Haptik auf, die mit dem optischen Teil gekoppelt ist. Die Intraokularlinse weist eine optische Achse beziehungsweise eine optische Hauptachse auf, die eine Vorderseite und eine Rückseite des optischen Teils durchdringt und insbesondere zentral mittig durchdringt. Die Haptik weist ein erstes Haptikteil auf, welches als erster Ring ausgebildet ist. Der erste Ring ist umlaufend um den optischen Teil ausgebildet. Die Haptik weist zumindest ein zweites Haptikteil auf, welches als zweiter Ring ausgebildet ist. Der zweite Ring ist umlaufend um den optischen Teil ausgebildet. Die beiden Haptikteile sind elastisch bewegbar. Zumindest einer der beiden Ringe ist in Umlaufrichtung um die optische Hauptachse betrachtet uneben ausgebildet. Dies bedeutet, dass der Ring entlang seiner Längsachse und somit entlang seiner Ringform betrachtet nicht in einer Ebene ausgebildet ist, sondern diesbezüglich sein Verlauf uneben ist. Zumindest einer der beiden Ringe weist genau zwei Ringtäler und genau zwei Ringberge auf. Durch eine derartige Ausgestaltung ist eine relativ einfache Geometrie dieses zumindest einen Haptikteils ausgebildet. Dies weist fertigungstechnische Vorteile auf. Darüber hinaus ist durch eine derartige Ausgestaltung die Halterung der Intraokularlinse im Kapselsack verbessert. Insbesondere kann sowohl eine unerwünschte Rotation der Intraokularlinse im Kapselsack als auch ein unerwünschtes Verkippen des optischen Teils zumindest reduziert werden. Damit kann eine verbesserte Positionsfixierung der Intraokularlinse in dem Kapselsack ermöglicht werden. Im Hinblick auf die genau zwei Ringtäler und die genau zwei Ringberge ist diese Höhenbezeichnung bezüglich einer Mittelebene des optischen Teils betrachtet. Diese Mittelebene ist senkrecht zur optischen Hauptachse orientiert und verläuft insbesondere mittig durch den optischen Teil. Die Ringtäler sind näher zu dieser Mittelebene angeordnet als die Ringberge.

Darüber hinaus ist durch eine derartige Ausgestaltung auch erreicht, dass die Intraokularlinse auch in axialer Richtung und somit in Richtung der optischen Hauptachse positionssicherer in dem Kapselsack positioniert werden kann.

In einer vorteilhaften Ausführung ist vorgesehen, dass der zumindest eine Ring mit den genau zwei Ringtälern und den genau zwei Ringbergen die Form eines Randrings beziehungsweise einer Umrandung einer Reitsattelform aufweist. Dies bedeutet, dass ausgehend von einer Reitsattelform der diesbezügliche Ring quasi eine Einfassung beziehungsweise einen Rand einer derartigen Reitsattelform darstellt. Dieser Ring kann somit auch als Reitsattelformrandring bezeichnet werden. Durch diese Spezifikation ist auch die Ausgestaltung mit zwei Ringtälern und genau zwei Ringbergen spezifiziert.

Die Form dieses Rings mit den genau zwei Ringtälern und genau zwei Ringbergen kann auch dahingehend verstanden werden, dass ein in einer Ebene gebildeter Ring um eine Achse, die senkrecht zur optischen Hauptachse steht, herumgekrümmt wird und somit diese dreidimensionale Form des Rings entsteht. Diese Achse, um welche dieser Ring herumgekrümmt ist, ist insbesondere in der Mittelebene des optischen Teils verlaufend.

Durch diese spezifische Form des zumindest einen Rings mit den genau zwei Ringtälern und den genau zwei Ringbergen kann den oben genannten Vorteilen verbessert Rechnung getragen werden. Es ist einerseits ein sehr einfacher Aufbau ermöglicht, andererseits dennoch eine gezielte punktuelle Verbindung mit dem optischen Teil ermöglicht. Dadurch ist eine hohe individuelle Verformbarkeit dieses Rings, insbesondere im Bereich der Ringberge, ermöglicht, andererseits eine ausreichende mechanische Verbindung mit dem optischen Teil geschaffen.

Insbesondere ist vorgesehen, dass der zumindest eine Ring mit seinen genau zwei Ringtälern an den optischen Teil mündet und mit den Ringbergen berührungslos zum optischen Teil angeordnet ist. Insbesondere mündet der Ring mit seinen genau zwei Ringtälern an zwei unterschiedlichen Stellen umfangsseitig an den optischen Teil. Durch eine derartige Ausgestaltung werden quasi nur zwei direkte mechanische Verbindungsstellen zwischen dem Ring und dem optischen Teil gebildet, nämlich durch die zwei gegenüberliegenden Ringtäler. Dadurch ist eine symmetrische mechanische Verbindungsausgestaltung zwischen dem Ring und dem optischen Teil geschaffen. Unerwünschte asymmetrische Verbindungen mit dem optischen Teil können dadurch vermieden werden. Durch die lediglich zwei vorgesehenen mechanischen Verbindungsstellen zwischen dem Ring und dem optischen Teil kann das Herstellen der Intraokularlinse vereinfacht werden. Andererseits ist dadurch die Formkomplexität der gesamten Intraokularlinse ebenfalls reduziert.

Die Intraokularlinse kann einstückig ausgebildet sein. Es kann aber auch vorgesehen sein, dass die Haptik und der optische Teil separate Komponenten sind. Insbesondere kann dann die Haptik mit dem optischen Teil mechanisch verbunden sein. Beispielsweise kann eine Klemmverbindung oder eine Steckverbindung oder eine Schnappverbindung zwischen einem Randbereich des optischen Teils und der Haptik ausgebildet sein. Beispielsweise kann die Haptik, insbesondere an den Ringtälern, an der Innenseite Vertiefungen aufweisen, in welche ein Randbereich des optischen Teils eingreift und gehalten ist. Es kann aber auch eine Klebeverbindung vorgesehen sein.

In einer vorteilhaften Ausführung ist vorgesehen, dass die Ringtäler bei einer Projektionsbetrachtung in Richtung der optischen Hauptachse in einem ersten Radius zur optischen Hauptachse angeordnet sind und die Ringberge bei dieser Projektionsbetrachtung in einem im Vergleich zum ersten Radius größeren zweiten Radius angeordnet sind. Bei dieser Projektionsbetrachtung ist der Ring in seiner Endposition somit nicht kreisförmig in der Projektionsebene gebildet, sondern diesbezüglich deformiert. Die Ringtäler sind in dem Zusammenhang weiter nach innen positioniert als die Ringberge. Die Ringberge sind somit radial weiter von der optischen Hauptachse entfernt als die Ringtäler. Dies gilt insbesondere für die Betrachtung in der Projektionsebene. Dadurch wird die elastische Bewegbarkeit des Rings gerade an den Ringbergen verbessert. Es kann somit eine individuellere Anpassung an die Ausgestaltung des Kapselsacks erfolgen und somit eine noch fein justiertere und präzisere Positionierung der Intraokularlinse im Kapselsack erreicht werden. Gerade durch diese radial weiter nach außen stehenden Ringberge, die dann auch frei auskragen und berührungslos zum optischen Teil ausgebildet sind und zu einer Mittelebene einen in Richtung der optischen Hauptachse betrachtet größeren Abstand als die Ringtäler aufweisen, sind diese Verformungsspezifikation und diese Verformungseigenschaften nochmals verbessert. Aufgrund dieses Vorteils kann auch die Verformungsanpassung an unterschiedlichste Ausgestaltungen von Kapselsäcken verbessert werden und dadurch wiederum die verbesserte Positionierung der Intraokularlinse in unterschiedlichen Kapselsäcken ermöglicht werden.

Vorzugsweise ist vorgesehen, dass der zumindest eine Ring mit den genau zwei Ringtälern und den genau zwei Ringbergen einen ersten Teilring aufweist, der sich von dem ersten Ringtal über einen ersten Ringberg bis zum nachfolgenden zweiten Ringtal erstreckt. Eine Lichte Weite des Teilrings, die sich zwischen den Ringtälern bemisst, ist mindestens 90 Prozent des Durchmessers des optischen Teils. Insbesondere beträgt diese Lichte Weite 100 Prozent oder etwas mehr als 100 Prozent dieses Durchmessers. Durch diese Ausgestaltung wird also ein Teilring geschaffen, der sich mit seiner Spannweite von dem ersten Ringtal zum zweiten Ringtal erstreckt und diesbezüglich einen einzigen U-förmigen Bogen darstellt. Dieser U-förmige Bogen fasst daher den optischen Teil in einem ersten halbseitigen Umfangsbereich ein beziehungsweise umspannt diesen optischen Teil. Auch dies ist insbesondere in der oben genannten Projektionsebene zu sehen.

Vorzugsweise sind zwei aufeinanderfolgende Ringtäler, die in Richtung der Längsachse des Rings an gegenüberliegenden Seiten zu einem Ringberg unmittelbar folgen, in Umlaufrichtung um die optische Hauptachse um 180° versetzt. Sie liegen somit in dieser azimutalen Richtung an gegenüberliegenden Seiten.

Vorzugsweise ist vorgesehen, dass der zumindest eine Ring mit den genau zwei Ringtälern und den genau zwei Ringbergen einen ersten Teilring aufweist, der sich von dem ersten Ringtal über einen ersten Ringberg bis zum nachfolgenden zweiten Ringtal erstreckt, wobei die Krümmung des ersten Teilrings bezüglich der optischen Hauptachse radial nach außen gerichtet ist. Die Krümmungsrichtung ist somit bei diesem ersten Teilring, der einen Halbring darstellt, entsprechend der Krümmung des Umfangs des optischen Teils ausgebildet, insbesondere über seine gesamte Länge. Diese Krümmungsrichtungen sind somit gleichgerichtet, wertmäßig jedoch unterschiedlich groß. Insbesondere ist ein Teilring als unebener, U-förmiger Bogen ausgebildet. Die U-Öffnung ist der optischen Hauptachse zugewandt.

Insbesondere betrifft dies die gesamte Bogenweite zwischen den beiden benachbarten Ringtälern dieses ersten Teilrings.

Vorzugsweise ist vorgesehen, dass die Ringberge in Richtung der optischen Hauptachse betrachtet einen größeren Abstand zur Mittelebene des optischen Teils aufweisen, als die Ringtäler. Insbesondere ist vorgesehen, dass der erste Ring die gleiche Form wie der zweite Ring aufweist. Insbesondere ist vorgesehen, dass diese beiden Ringe symmetrisch zu der Mittelebene des optischen Teils angeordnet sind. Dies bedeutet, dass die Ringtäler insbesondere direkt aneinander anliegen und die Ringberge an der jeweils gleichen azimutalen Position in Umlaufrichtung um die optische Hauptachse angeordnet sind und maximal beabstandet sind. Dies bedeutet, dass ein Abstand, der in Richtung der optischen Hauptachse bemessen ist, in Umlaufrichtung um die optische Hauptachse variiert und an den Positionen von Ringtälern von diesen beiden Ringen minimal ist und an Positionen der Ringberge der beiden Ringe maximal ist.

Der optische Teil weist insbesondere eine Mittendicke auf. Diese Mittendicke ist entlang der optischen Hauptachse bemessen und stellt die größte Dicke des optischen Teils dar. Es ist eine Dicke des optischen Teils gebildet, die sich zwischen dem Maximum der gekrümmten Vorderseite und/oder dem Maximum der gekrümmten Rückseite des optischen Teils und der Mittelebene des optischen Teils entlang der optischen Hauptachse betrachtet bemisst. Insbesondere ist diese Dicke die Hälfte der Mittendicke des optischen Teils. Insbesondere ist im nicht implantierten Zustand der Intraokularlinse ein Abstand eines Ringbergs zur Mittelebene, der somit senkrecht zur Mittelebene bemessen ist und somit in Richtung der optischen Hauptachse betrachtet ist, größer als diese Dicke. Dadurch ist in besonders vorteilhafter Weise erreicht, dass insbesondere eine Oberseite, insbesondere die Rückseite des optischen Teils im implantierten Zustand im Auge beabstandet zur einer Kapselsackwand, insbesondere zur hinteren Kapselsackwand, positioniert werden kann.

Vorzugsweise ist vorgesehen, dass die beiden Haptikringe in Umlaufrichtung um die optische Hauptachse an gleicher Azimutposition mit ihren beiden Ringtälern angeordnet sind. Insbesondere sind die beiden Haptikringe beziehungsweise der erste Ring und der zweite Ring in Umlaufrichtung um die optische Hauptachse an gleicher Azimutposition mit ihren beiden Ringbergen angeordnet.

Vorzugsweise ist vorgesehen, dass die Ringe so zueinander angeordnet sind, dass sie im Bereich der Ringberge ein radial nach außen gerichtetes geöffnetes Maul beziehungsweise eine Maulform darstellen. Dieses Maul hat in radialer Richtung betrachtet am radial äußersten Ende die größte Maulöffnung. Diese beiden Ringe nehmen ausgehend von einem jeweiligen Ringtal in Umlaufrichtung um die optische Hauptachse betrachtet bis hin zum anderen nächsten Ringtal im Radius zu und der Abstand dieser beiden Ringe nimmt dabei in Richtung der optischen Hauptachse betrachtet bis zu den beiden Ringbergen kontinuierlich zu und nimmt dann von den Ringbergen bis zu dem jeweils nachfolgenden zweiten Ringtal wieder ab.

In einer vorteilhaften Ausführung ist vorgesehen, dass die Ringe mit ihren jeweiligen Ringtälern an einer Umfangswand des optischen Teils direkt mit dem optischen Teil verbunden sind, insbesondere einstückig damit ausgebildet sind. Es kann vorgesehen sein, dass die benachbarten Ringtäler der beiden Ringe an dieser spezifischen Azimutposition benachbart und überlappungsfrei angeordnet sind oder überlappend miteinander ausgebildet sind. Die Ringe können auch einstückig miteinander als Ringensemble ausgebildet sein.

In einer vorteilhaften Ausführung kann vorgesehen sein, dass die Ringe in ihrem in eine Ebene aufgeklappten Zustand, in dem dann nur die zweidimensionale Formgebung dargestellt ist, als Kreisringe ausgebildet sind. Sie können bei einer diesbezüglichen Betrachtung im Zweidimensionalen in einer Ebene auch als Ovalringe ausgebildet sein.

Vorzugsweise ist zumindest einer der beiden Ringe in Umlaufrichtung unterbrechungsfrei und somit vollständig geschlossen ausgebildet. In einer vorteilhaften Ausführung ist vorgesehen, dass ein axialer Abstand eines Ringbergs eines Rings zur Mittelebene des optischen Teils größer ist, als ein Abstand eines, insbesondere mittigen, Wölbungsmaximums des optischen Teils zur Mittelebene. Dadurch ist es ermöglicht, dass die Intraokularlinse auch so im Kapselsack positioniert ist, dass der optische Teil beabstandet zu einer Kapselsackwand angeordnet ist. Dies ist insbesondere von Vorteil für eine Rückseite des optischen Teils. Diese Rückseite kann dann im Kapselsack beabstandet zu einer hinteren Kapselsackwand angeordnet werden. Dadurch kann Kammerwasser zwischen dieser Rückseite und dem hinteren Kapselsack gelangen. Dadurch kann die Migration von Zellen vermieden werden und somit eine Eintrübung des hinteren Kapselsacks vermieden werden.

Insbesondere ist die Intraokularlinse eine Hinterkammerlinse zum Implantieren in einen Kapselsack eines Auges.

Die diesbezüglich vorgeschlagene Intraokularlinse ist faltbar und kann symmetrisch im Kapselsack positioniert werden, ohne dass es zu axialen Verschiebungen kommt. Die diesbezüglich spezifizierte Haptik kann sich in verbesserter Weise den räumlichen Gegebenheiten des Kapselsacks individuell anpassen. Dadurch können unerwünschte Lageveränderungen des Optikteils beziehungsweise des optischen Teils der Intraokularlinse vermieden werden. Insbesondere ist durch diese Ausgestaltung der Haptik auch eine verbesserte Rotationsstabilität der Intraokularlinse im Kapselsack erreicht.

Insbesondere ist die Intraokularlinse einstückig aus einem Polymermaterial ausgebildet.

Insbesondere ist die Intraokularlinse als kapselsackimplantierende Intraokularlinse ausgebildet. Sie kann in dem Zusammenhang auch als Kapselsack-Implantations-Intraokularlinse bezeichnet werden. Insbesondere ist sie in dem Zusammenhang eine Hinterkammerlinse zum Implantieren in einen Kapselsack eines Auges. Dies bedeutet, dass die Intraokularlinse bestimmungsgemäß, insbesondere nur, zur Implantation in einen Kapselsack eines Auges vorgesehen ist.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1: eine perspektivische Darstellung eines Ausführungsbeispiels einer erfindungsgemäßen Intraokularlinse;
- Fig. 2: eine perspektivische Darstellung eines Ausführungsbeispiels einer Haptik für eine Intraokularlinse;
- Fig. 3: eine schematische Darstellung der Intraokularlinse gemäß Fig. 1 in einer Draufsicht in Richtung der optischen Hauptachse der Intraokularlinse;
- Fig. 4: eine schematische Darstellung eines Rings der Haptik in einer zweidimensionalen Grundform, in welcher der Ring ein Kreisring ist;
- Fig. 5: eine Darstellung gemäß Fig. 4, in welcher ein weiteres Ausführungsbeispiel eines Rings in einer Grundform als Ovalring dargestellt ist.
- Fig. 6a: eine schematische Darstellung eines Ausführungsbeispiels einer Intraokularlinse, die in einen Kapselsack implantiert ist;
- Fig. 6b: eine Darstellung gemäß Fig. 6a, in welcher die Intraokularlinse in einen zu Fig. 6a größeren Kapselsack implantiert ist; und
- Fig. 6c: eine Darstellung gemäß Fig. 6a und 6b, in welcher die Intraokularlinse in einen nochmals größeren Kapselsack implantiert ist.

### Bevorzugte Ausführungsbeispiele der Erfindung

In den Figuren werden gleiche oder funktionsgleiche Elemente mit den gleichen Bezugszeichen versehen.

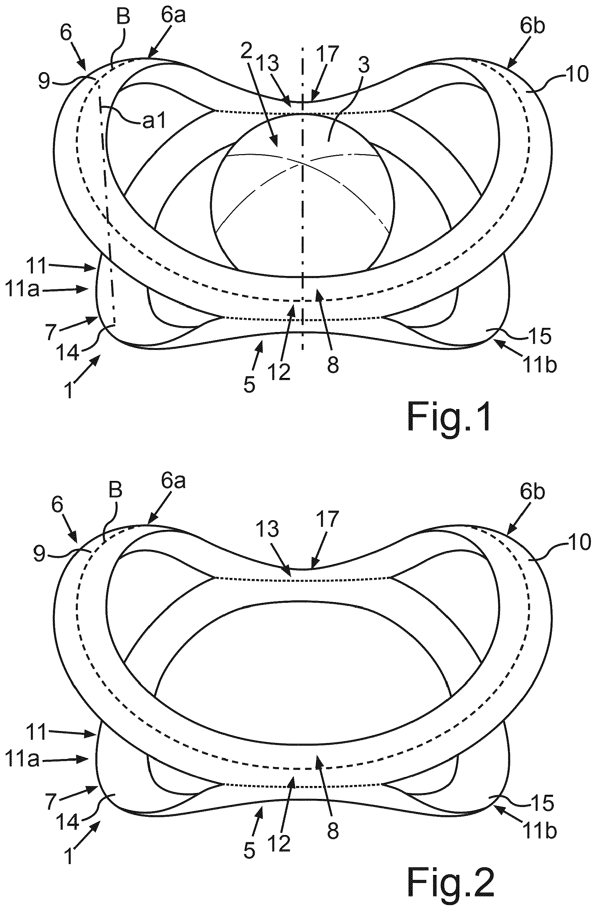

In Fig. 1 ist in einer perspektivischen Darstellung ein Ausführungsbeispiel einer künstlichen Intraokularlinse 1 gezeigt. Diese Intraokularlinse 1 ist eine Hinterkammerlinse zum Implantieren in einen Kapselsack eines Auges. Sie kann daher auch als Kapselsack-Implantations-Intraokularlinse bezeichnet werden. Die Intraokularlinse 1 weist einen optischen Teil 2 auf. Der optische Teil 2 ist als Linse ausgebildet. Er ist zur Erzeugung einer definierten optischen Abbildungseigenschaft der Intraokularlinse 1 ausgebildet. Die Intraokularlinse 1 weist eine optische Achse bzw. eine optische Hauptachse A auf. Diese durchdringt eine Vorderseite 3 des optischen Teils 2 und eine Rückseite 4 des optischen Teils 2 zentral und mittig des optischen Teils 2.

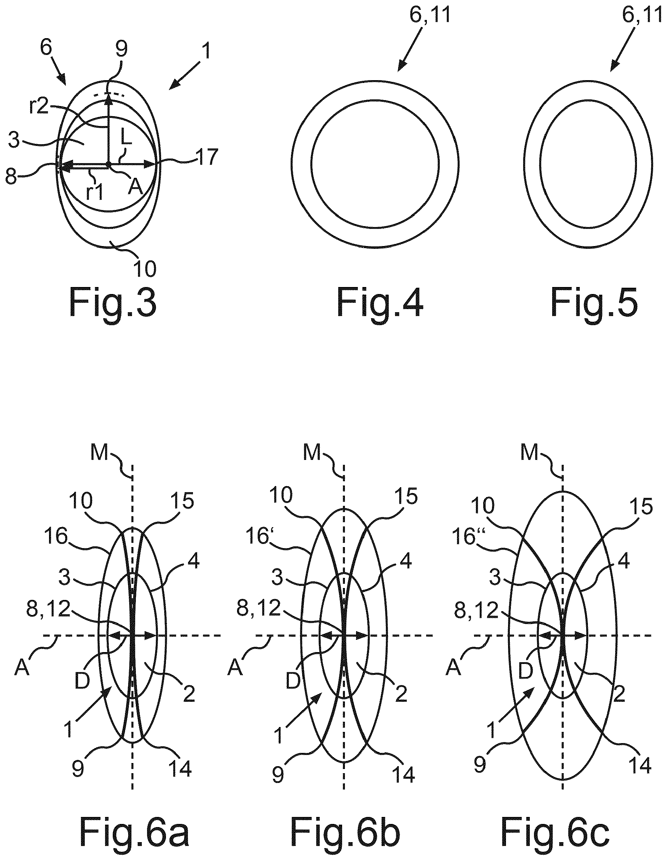

Die Intraokularlinse 1 weist eine Haptik 5 auf. Die Haptik 5 ist im gezeigten Ausführungsbeispiel aus zwei Ringen 6 und 7 gebildet. Der erste Ring 6 ist im Ausführungsbeispiel zumindest bereichsweise elastisch verformbar. Der erste Ring 6 ist in Umlaufrichtung um die optische Hauptachse A uneben ausgebildet. Dies bedeutet, dass der erste Ring 6 mit seiner gesamten Formgebung beziehungsweise seiner gesamten Geometrie nicht in einer einzigen Ebene angeordnet ist. Vielmehr ist der erste Ring 6 als dreidimensional geformter Ring gebildet. Bezüglich seiner dreidimensionalen Formgebung weist der erste Ring 6 genau zwei Ringtäler 8 und 17 auf. Der erste Ring 6 weist darüber hinaus genau zwei Ringberge 9 und 10 auf. Die Ringtäler 8 und 17 sowie die Ringberge 9 und 10 sind insbesondere in Bezug zu einer Mittelebene M (Fig. 6a bis 6c) des optischen Teils 2 zu sehen. Diese Mittelebene M des als Linse ausgebildeten optischen Teils 2 verläuft insbesondere mittig durch diesen optischen Teil 2 und ist senkrecht zur optischen Hauptachse A orientiert. In Richtung der optischen Hauptachse A betrachtet weisen die beiden Ringtäler 8 und 17 somit einen geringeren Abstand zur Mittelebene M als die Ringberge 9 und 10 auf. Insbesondere ist vorgesehen, dass die Ringtäler 8 und 17 in Umlaufrichtung um die optische Hauptachse A um 180° versetzt zueinander angeordnet sind. Entsprechendes ist vorzugsweise für die Ringberge 9 und 10 ausgebildet. Die Ringberge 9 und 10 sind entlang der Längsachse B des ersten Rings 9 im gleichen Abstand zu den beiden Ringtälern 8 und 17 ausgebildet.

Insbesondere weist der erste Ring 6 die Form eines Randrings einer Reitsattelform auf. Der erste Ring 6 ist in seiner Position in dem fertigen Endzustand der Intraokularlinse 1 als Kreisring oder Ovalring ausgebildet, der um eine Achse, die in der Mittelebene M liegt und senkrecht zur optischen Hauptachse A orientiert ist, herum gekrümmt ist. Dadurch entsteht diese Reitsattelform. Es kann auch vorgesehen sein, dass diese Endposition des ersten Rings 6 ein ringförmiger Ausschnitt aus einer Mantelwand eines Hohlzylinders gebildet ist, wobei dieser Hohlzylinder als Zylinderachse diejenige aufweist, die in der Mittelebene M liegt und senkrecht zur optischen Hauptachse A orientiert ist.

Der erste Ring 6 ist mit seinen Ringtälern 8 und 17 umfangsseitig an dem optischen Teil 2 gekoppelt. Er ist insbesondere direkt mit dem optischen Teil 2 verbunden. Der erste Ring 6 ist mit seinen Ringbergen 9 und 10 berührungslos zum optischen Teil 2 angeordnet. Diese Ringberge 9 und 10 sind in radialer Richtung zur optischen Hauptachse A betrachtet somit radial nach außen frei auskragend.

Wie in dem Beispiel in Fig. 1 des Weiteren zu erkennen ist, weist die Haptik 5 im Ausführungsbeispiel einen zweiten Ring 11 auf. Dieser zweite Ring 11 ist ebenfalls in Umlaufrichtung um die optische Hauptachse A unterbrechungsfrei ausgebildet und vollständig umlaufend um die optische Hauptachse A ausgebildet. Insbesondere ist vorgesehen, dass dieser zweite Ring 11 ebenfalls genau zwei Ringtäler 12 und 13 sowie zwei Ringberge 14 und 15 aufweist. Der zweite Ring 11 ist vorzugsweise so zum ersten Ring 6 angeordnet, dass in Umlaufrichtung um die optische Hauptachse A die beiden Ringtäler 8 und 12 an gleicher Azimutposition angeordnet sind und auch die beiden Ringtäler 17 und 13 in gleicher Azimutposition angeordnet sind. Insbesondere sind die beiden Ringe 6 und 11 so ausgebildet und angeordnet, dass die Ringberge 9 und 14 an gleicher Azimutposition angeordnet sind und die Ringberge 10 und 15 an gleicher Azimutposition angeordnet sind.

Im Übrigen gelten bezüglich der Form und Anordnung relativ zum optischen Teil 2 für den zweiten Ring 11 die gleichen Ausführungen, wie sie zum ersten Ring 6 dargelegt wurden.

In Fig. 2 ist in einer perspektivischen Darstellung die Haptik 5 der Intraokularlinse 1 ohne den optischen Teil 2 gezeigt. Die Haptik 5 mit den beiden Ringen 6 und 7 ist hier einstückig ausgebildet. Die gestrichelten Trennlinien sind hier lediglich als die jeweilige Geometrie der Ringe 6 und 7 spezifizierende Hilfslinien zu verstehen. Die Ringe 6 und 7 können auch separat ausgebildet ein.

In einer Draufsicht, wie sie beispielhaft in Fig. 3 schematisch gezeigt ist und bei welcher in Richtung der optischen Hauptachse A auf die Intraokularlinse 1 geblickt wird, ist zu erkennen, dass die Ringtäler 8 und 17 des ersten Rings 6 in einem ersten Radius r1 zur optischen Hauptachse A angeordnet sind. Dieser Radius r1 ist kleiner als ein Radius r2. Dieser zweite Radius r2 stellt den radialen Abstand der Ringberge 9 und 10 zur optischen Hauptachse A dar. Die Ringberge 9 und 10 des ersten Rings 6 sind somit radial weiter von der optischen Hauptachse A entfernt als die Ringtäler 8 und 17. Gleiches gilt für die Ringtäler 12 und 13 im Vergleich zu den Ringbergen 14 und 15 des zweiten Rings 11.

Insbesondere ist vorgesehen, dass der erste Ring 6 einen ersten Teilring 6a aufweist, der sich von dem ersten Ringtal 8 über den ersten Ringberg 9 bis zum zweiten Ringtal 17 erstreckt. Die Krümmung beziehungsweise die Krümmungsrichtung dieses ersten Teilrings 6a ist bezüglich der optischen Hauptachse A radial nach außen gerichtet. Der erste Teilring 6a stellt somit in der Projektionsbetrachtung gemäß Fig. 3 einen U-Bogen dar. Er erstreckt sich von einer Umfangsstelle des optischen Teils 2 bis zu einer weiteren Umfangsstelle des optischen Teils 2, die diesbezüglich um 180° versetzt dazu ist. Entsprechendes gilt für einen zweiten Teilring 6b des ersten Rings 6. Die beiden Teilringe 6a und 6b ergeben zusammen den ersten Ring 6. Gleiches gilt für einen ersten Teilring 11a und einen zweiten Teilring 11b des zweiten Rings 11.

Eine Lichte Weite L, die sich zwischen den Ringtälern 8 und 17 geradlinig erstreckt und durch die optische Hauptachse A verläuft, bemisst sich auf mindestens 90 Prozent, insbesondere zumindest 100 Prozent des Durchmessers des optischen Teils 2. Selbiges gilt für eine Lichte Weite zwischen den Ringtälern 12 und 13.

Dieser erste Teilring 6a ist bezüglich seiner Krümmung zur optischen Hauptachse A radial nach außen gerichtet. Gleiches gilt für den zweiten Teilring 6b. Entsprechendes gilt für die Krümmungen beziehungsweise Krümmungsrichtungen der Teilringe 11a und 11b.

Die Ringberge 9 und 14 weisen in einem nicht implantierten Grundzustand der Intraokularlinse 1 einen Abstand a1 auf, der in Richtung der optischen Hauptachse A bemessen ist. Dieser Abstand a1 ist größer als ein in Richtung der optischen Hauptachse A bemessener, gegebenenfalls vorhandener Abstand zwischen den Ringtälern 8 und 12 und/oder den Ringtälern 17 und 13. Insbesondere ist ein entsprechender Abstand a1 auch zwischen den Ringbergen 10 und 15 ausgebildet. In Umlaufrichtung um die optische Hauptachse A betrachtet variiert somit ein Abstand zwischen den beiden Ringen 6 und 11 von einem Minimum beziehungsweise einem Abstand 0 an den Ringtälern 8, 12 und 17, 13 bis zu einem jeweiligen Maximum, welches durch die Abstände a1 zwischen den Ringbergen 9, 14 und/oder den Ringbergen 10, 15 gebildet ist.

Darüber hinaus ist vorgesehen, dass die Ringberge 9 und 10 des ersten Rings 6 in Richtung der optischen Hauptachse A betrachtet einen größeren Abstand zur Mittelebene M im optischen Teil 2 aufweisen, als die Ringtäler 8 und 17. Insbesondere beträgt dieser Abstand die Hälfte des Abstands a1. Insbesondere gilt Entsprechendes für die Abstände der Ringberge 14 und 15 zu dieser Mittelebene M, insbesondere in Bezug zu den Ringtälern 12 und 13.

Insbesondere sind die beiden Ringe 6 und 11 symmetrisch zur Mittelebene M ausgebildet.

In Fig. 4 ist ein Ausführungsbeispiel für einen ersten Ring 6 gezeigt. Dieser Ring 6 ist in der Figurenebene dargestellt und somit im Vergleich zu Fig. 1 diesbezüglich aufgeklappt und somit eben dargestellt. Er weist in dieser aufgeklappten ebenen Darstellung die Form eines Kreisrings auf. In Fig. 5 ist dazu in einer alternativen Ausgestaltung der erste Ring 6 in dieser Figurenebene gezeigt und somit im Vergleich zu der Endposition einer Intraokularlinse 1 in einem aufgeklappten Zustand und somit in einer zweidimensionalen Darstellung gezeigt. Diese aufgeklappte Grundform kann hier in der Draufsicht ein Ovalring sein. Entsprechende Formen, wie sie in der zweidimensionalen aufgeklappten Grundform des ersten Rings 6 in Fig. 4 und Fig. 5 gezeigt sind, können auch für den zweiten Ring 11 ausgebildet sein.

In Fig. 6a ist in einer schematischen Darstellung ein implantierter Zustand der Intraokularlinse 1 in einen Kapselsack 16 gezeigt. Der hier relativ kleine Kapselsack 16 bedingt, dass die Ringberge 9, 10, 14 und 15 relativ stark einander zubewegt sind und somit elastisch diesbezüglich verformt sind. Der optische Teil 2 weist eine Mittendicke D auf. Diese Mittendicke D ist entlang der optischen Hauptachse A bemessen und stellt die größte Dicke des optischen Teils 2 dar. Eine Dicke, die sich zwischen der Vorderseite 3 und der Mittelebene M entlang der optischen Hauptachse A bemisst, stellt die Hälfte dieser Mittendicke D dar. Insbesondere ist im nicht implantierten Zustand der Intraokularlinse 1 ein Abstand eines Ringbergs 9, 10 zur Mittelebene M in Richtung der optischen Hauptachse A betrachtet größer als dieser Abstand D/2.

Insbesondere ist es durch diese Ausgestaltung auch ermöglicht, dass die Intraokularlinse 1 im implantierten Zustand im Kapselsack 6 mit ihrem optischen Teil 2 beabstandet zu den Kapselsackwänden, insbesondere einer hinteren Kapselsackwand, angeordnet werden kann. Damit kann Kammerwasser zwischen einer Rückseite 4 und der hinteren Kapselsackwand des Kapselsacks 16 gelangen.

In Fig. 6b ist in einem weiteren Ausführungsbeispiel der implantierte Zustand der Intraokularlinse 1 in einen Kapselsack 16' gezeigt. Dieser Kapselsack 16' ist größenmäßig und/oder formmäßig unterschiedlich zum Kapselsack 16. Aufgrund der Elastizität und spezifischen Formgebung der Haptik 5 ist auch darin eine positionssichere und/oder rotationsstabile und/oder kippfreie Positionierung ermöglicht. Eine weitere Darstellung eines implantierten Zustands der Intraokularlinse 1 in einen diesbezüglich nochmals unterschiedlichen Kapselsack 16" ist in Fig. 6c schematisch gezeigt.

## Patentansprüche

1. Intraokularlinse (1) mit einem einzigen optischen Teil (2) und mit einer Haptik (5), die mit dem optischen Teil (2) gekoppelt ist, und mit einer optischen Hauptachse (A), die eine Vorderseite (3) und eine Rückseite (4) des optischen Teils (2) durchdringt, wobei die Haptik (5) ein erstes Haptikteil aufweist, welches als erster Ring (6) und umlaufend um den optischen Teil (2) ausgebildet ist, und zumindest ein zweites Haptikteil aufweist, welches als zweiter Ring (11) und umlaufend um den optischen Teil (2) ausgebildet ist und welches relativ zum ersten Haptikteil elastisch bewegbar ist, wobei zumindest einer der beiden Ringe (6, 11) in Umlaufrichtung um die optische Hauptachse (A) uneben ausgebildet ist,
**dadurch gekennzeichnet, dass**
zumindest einer der beiden Ringe (6, 11) genau zwei Ringtäler (8, 17, 12, 13) und genau zwei Ringberge (9, 10, 14, 15) aufweist.

2. Intraokularlinse (1) nach Anspruch 1,
**dadurch gekennzeichnet, dass**
der zumindest eine Ring (6, 11) mit den genau zwei Ringtälern (8, 17 12, 13) und genau zwei Ringbergen (9, 10, 14, 15) die Form eines Randrings einer Reitsattelform aufweist.

3. Intraokularlinse (1) nach Anspruch 1 oder 2,
**dadurch gekennzeichnet, dass**
der zumindest eine Ring (6, 11) mit seinen Ringtälern (8, 17, 12, 13) umfangsseitig an den optischen Teil (2) mündet und mit den Ringbergen (9, 10, 14, 15) berührungslos zum optischen Teil (2) angeordnet ist.

4. Intraokularlinse (1) nach einem der vorhergehenden Ansprüche,
**dadurch gekennzeichnet, dass**
die Ringtäler (8, 17, 12, 13) bei einer Projektionsbetrachtung in Richtung der optischen Hauptachse (A) in einem ersten Radius (r1) zur optischen Hauptachse (A) angeordnet sind und die Ringberge (9, 10, 14, 15) bei dieser Projektionsbetrachtung in einem im Vergleich zum ersten Radius (r1) größeren zweiten Radius (r2) angeordnet sind.

5. Intraokularlinse (1) nach einem der vorhergehenden Ansprüche,
**dadurch gekennzeichnet, dass**
der zumindest eine Ring (6, 11) mit den genau zwei Ringtäler (8, 17, 12, 13) und genau zwei Ringbergen (9, 10, 14, 15) einen ersten Teilring (6a, 6b, 11a, 11b) aufweist, der sich von dem ersten Ringtal (8, 12) über einen ersten Ringberg (9, 10, 14, 15) bis zum zweiten Ringtal (17, 13) erstreckt, wobei eine Lichte Weite (L) des Teilrings (6a, 6b, 11a, 11b), die zwischen den Ringtäler (8, 17, 12, 13) bemessen ist, mindestens 90% des Durchmessers des optischen Teils (2) beträgt.

6. Intraokularlinse (1) nach einem der vorhergehenden Ansprüche,
**dadurch gekennzeichnet, dass**
der zumindest eine Ring (6, 11) mit den genau zwei Ringtäler (8, 17, 12, 13) und genau zwei Ringbergen (9, 10, 14, 15) einen ersten Teilring (6a, 6b, 11a, 11b) aufweist, der sich von dem ersten Ringtal (8, 12) über einen ersten Ringberg (9, 10, 14, 15) bis zum zweiten Ringtal (17, 13) erstreckt, wobei die Krümmung des ersten Teilrings (6a, 6b, 11a, 11b) bezüglich der optischen Hauptachse (A) radial nach außen gerichtet ist.

7. Intraokularlinse (1) nach einem der vorhergehenden Ansprüche,
**dadurch gekennzeichnet, dass**
die Ringberge (9, 10, 14, 15) parallel zur optischen Hauptachse (A) betrachtet einen größeren Abstand zur Mittelebene (M) des optischen Teils (2) aufweisen, als die Ringtäler (8, 17, 12, 13).

8. Intraokularlinse (1) nach einem der vorhergehenden Ansprüche,
**dadurch gekennzeichnet, dass**
der erste Ring (6) die gleiche Form wie der zweite Ring (11) aufweist, und die Ringe (6, 11) symmetrisch zu der Mittelebene (M) des optischen Teils (2) angeordnet sind.

9. Intraokularlinse (1) nach Anspruch 8,
**dadurch gekennzeichnet, dass**
die Ringe (6, 11) mit ihren jeweiligen Ringtälern (8, 17, 12, 13) an einer Umfangswand des optischen Teils (2) direkt mit dem optischen Teil (2) verbunden sind, insbesondere einstückig damit ausgebildet sind.

10. Intraokularlinse (1) nach einem der vorhergehenden Ansprüche,
**dadurch gekennzeichnet, dass**
die Intraokularlinse (1) eine Hinterkammerlinse zum Implantieren in einen Kapselsack (16, 16', 16") eines Auges ist.

## Claims

1. Intraocular lens (1) comprising a single optical part (2) and comprising a haptic (5) coupled to the optical part (2), and having a main optical axis (A) which passes through a front side (3) and a back side (4) of the optical part (2), the haptic (5) comprising a first haptic part which is in the form of a first ring (6) and encircles the optical part (2), and at least one second haptic part which is in the form of a second ring (11) and encircles the optical part (2) and which is elastically movable relative to the first haptic part, at least one of the two rings (6, 11) having an uneven form in the circumferential direction about the main optical axis (A),
**characterized in that**
at least one of the two rings (6, 11) has exactly two ring valleys (8, 17, 12, 13) and exactly two ring peaks (9, 10, 14, 15).

2. Intraocular lens (1) according to Claim 1,
**characterized in that**
the at least one ring (6, 11) with the exactly two ring valleys (8, 17, 12, 13) and exactly two ring peaks (9, 10, 14, 15) has the shape of an edge ring of a saddle form.

3. Intraocular lens (1) according to Claim 1 or 2, **characterized in that**
the at least one ring (6, 11) merges with the optical part (2) with its ring valleys (8, 17, 12, 13) along the circumferential side and its ring peaks (9, 10, 14, 15) are arranged so as not to be in contact with the optical part (2).

4. Intraocular lens (1) according to any one of the preceding claims,
**characterized in that**
the ring valleys (8, 17, 12, 13) are arranged at a first radius (r1) with respect to the main optical axis (A) in the case of a projected view in the direction of the main optical axis (A) and the ring peaks (9, 10, 14, 15) are arranged at a second radius (r2) which is comparatively larger than the first radius (r1) in the case of this projected view.

5. Intraocular lens (1) according to any one of the preceding claims,
**characterized in that**
the at least one ring (6, 11) with the exactly two ring valleys (8, 17, 12, 13) and exactly two ring peaks (9, 10, 14, 15) has a first ring portion (6a, 6b, 11a, 11b) which extends from the first ring valley (8, 12) over a first ring peak (9, 10, 14, 15) and up to the second ring valley (17, 13), a clear width (L) of the ring portion (6a, 6b, 11a, 11b) as measured between the ring valleys (8, 17, 12, 13) being at least 90% of the diameter of the optical part (2).

6. Intraocular lens (1) according to any one of the preceding claims,
**characterized in that**
the at least one ring (6, 11) with the exactly two ring valleys (8, 17, 12, 13) and exactly two ring peaks (9, 10, 14, 15) has a first ring portion (6a, 6b, 11a, 11b) which extends from the first ring valley (8, 12) over a first ring peak (9, 10, 14, 15) and up to the second ring valley (17, 13), the curvature of the first ring portion (6a, 6b, 11a, 11b) being directed radially to the outside in relation to the main optical axis (A).

7. Intraocular lens (1) according to any one of the preceding claims,
**characterized in that**
the ring peaks (9, 10, 14, 15) have a greater distance from the central plane (M) of the optical part (2) than the ring valleys (8, 17, 12, 13) when considered parallel to the main optical axis (A).

8. Intraocular lens (1) according to any one of the preceding claims,
**characterized in that**
the first ring (6) has the same shape as the second ring (11) and the rings (6, 11) are arranged symmetrically in relation to the central plane (M) of the optical part (2) .

9. Intraocular lens (1) according to Claim 8,
**characterized in that**
the rings (6, 11), by way of their respective ring valleys (8, 17, 12, 13), are directly connected to the optical part (2), in particular formed in one piece therewith, at a circumferential wall of the optical part (2).

10. Intraocular lens (1) according to any one of the preceding claims,
**characterized in that**
the intraocular lens (1) is a posterior chamber lens for implantation into a capsular bag (16, 16', 16") of an eye.

## Revendications

1. Lentille intraoculaire (1) comprenant une partie optique (2) unique et une structure haptique (5) qui est accouplée à la partie optique (2) ainsi qu'un axe optique principal (A) qui traverse une face avant (3) et une face arrière (4) de la partie optique (2), la structure haptique (5) comportant une première partie haptique réalisée sous la forme d'un premier anneau (6) entourant la partie optique (2), et au moins une deuxième partie haptique réalisée sous la forme d'un deuxième anneau (11) entourant la partie optique (2) et pouvant être déplacé élastiquement par rapport à la première partie haptique, au moins l'un des deux anneaux (6, 11) étant réalisé de manière irrégulière dans la direction périphérique autour de l'axe optique principal (A),
**caractérisé en ce qu'**au moins l'un des deux anneaux (6, 11) comporte précisément deux vallées d'anneau (8, 17, 12, 13) et précisément deux montagnes d'anneau (9, 10, 14, 15) .

2. Lentille intraoculaire (1) selon la revendication 1, **caractérisée en ce que**
ledit au moins un anneau (6, 11) comportant les précisément deux vallées annulaires (8, 17 12, 13) et précisément deux montagnes annulaires (9, 10, 14, 15) présente la forme d'un anneau de bordure d'une forme de selle de cheval.

3. Lentille intraoculaire (1) selon la revendication 1 ou 2, **caractérisée en ce que**
ledit au moins un anneau (6, 11) débouche avec ses vallées annulaires (8, 17, 12, 13) du côté périphérique sur la partie optique (2) et est disposé de manière à ce que les montagnes annulaires (9, 10, 14, 15) ne soient pas en contact avec la partie optique (2).

4. Lentille intraoculaire (1) selon l'une des revendications précédentes, **caractérisée en ce que**
les vallées annulaires (8, 17, 12, 13) sont disposées selon un premier rayon (r1) par rapport à l'axe optique principal (A) lorsqu'elles sont observées en projection dans la direction de l'axe optique principal (A) et **en ce que** les montagnes annulaires (9, 10, 14, 15) sont disposées selon un deuxième rayon (r2) plus grand par comparaison au premier rayon (r1) lors de cette observation en projection.

5. Lentille intraoculaire (1) selon l'une des revendications précédentes, **caractérisée en ce que**
ledit au moins un anneau (6, 11) comportant les précisément deux vallées annulaires (8, 17, 12, 13) et précisément deux montagnes annulaires (9, 10, 14, 15) comporte un premier anneau partiel (6a, 6b, 11a, 11b) s'étendant de la première vallée annulaire (8, 12) à la deuxième vallée annulaire (17, 13) en passant par une première montagne annulaire (9, 10, 14, 15), une largeur intérieure (L) de l'anneau partiel (6a, 6b, 11a, 11b), qui est mesurée entre les vallées annulaires (8, 17, 12, 13), étant au moins égale à 90 % du diamètre de la partie optique (2).

6. Lentille intraoculaire (1) selon l'une des revendications précédentes, **caractérisée en ce que**
ledit au moins un anneau (6, 11) comportant les précisément deux vallées annulaires (8, 17, 12, 13) et précisément deux montagnes annulaires (9, 10, 14, 15) comporte un premier anneau partiel (6a, 6b, 11a, 11b) s'étendant de la première vallée annulaire (8, 12) à la deuxième vallée annulaire (17, 13) en passant par une première montagne annulaire (9, 10, 14, 15), la courbure du premier anneau partiel (6a, 6b, 11a, 11b) étant dirigée radialement vers l'extérieur par rapport à l'axe optique principal (A).

7. Lentille intraoculaire (1) selon l'une des revendications précédentes, **caractérisée en ce que**
les montagnes annulaires (9, 10, 14, 15), observées parallèlement à l'axe optique principal (A), présentent une distance plus grande par rapport au plan médian (M) de la partie optique (2) que les vallées annulaires (8, 17, 12, 13).

8. Lentille intraoculaire (1) selon l'une des revendications précédentes, **caractérisée en ce que**
le premier anneau (6) présente la même forme que le deuxième anneau (11), et **en ce que** les anneaux (6, 11) sont disposés symétriquement par rapport au plan médian (M) de la partie optique (2).

9. Lentille intraoculaire (1) selon la revendication 8, **caractérisée en ce que**
les anneaux (6, 11) sont reliés directement à la partie optique (2) par leurs vallées annulaires respectives (8, 17, 12, 13) au niveau d'une paroi périphérique de la partie optique (2), et sont en particulier formés d'un seul tenant avec celle-ci.

10. Lentille intraoculaire (1) selon l'une des revendications précédentes, **caractérisée en ce que**
la lentille intraoculaire (1) est une lentille de chambre postérieure destinée à être implantée dans un sac capsulaire (16, 16', 16") d'un œil.
